(19) Europäisches Patentamt
European Patent Office
Office européen des brevets

(11) **EP 4 548 904 A1**

(12) **EUROPEAN PATENT APPLICATION**

(43) Date of publication:
**07.05.2025   Bulletin 2025/19**

(21) Application number: **24195519.4**

(22) Date of filing: **21.08.2024**

(51) International Patent Classification (IPC):
*A61K 8/02* (2006.01)       *A61K 8/73* (2006.01)
*C09D 105/08* (2006.01)     *C09D 133/08* (2006.01)
*C09D 179/00* (2006.01)     *C09D 191/00* (2006.01)
*C09D 179/02* (2006.01)

(52) Cooperative Patent Classification (CPC):
(C-Sets available)
**C09D 105/08; A61K 8/0241; A61K 8/731;
A61Q 1/10; C08J 3/126; C08J 3/128; C09D 133/08;
C09D 179/02; C09D 191/00;** A61K 2800/412;
C08J 2301/10; C08J 2301/12; C08J 2401/28;
C08J 2433/08; C08J 2479/02;               (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:   **31.10.2023   JP 2023187201**

(71) Applicant: **Fujifilm Business Innovation Corp.
Tokyo 107-0052 (JP)**

(72) Inventors:
• **YOSHIDA, Kazusei
Minamiashigara (JP)**
• **NAITO, Ayu
Minamiashigara (JP)**
• **KASHIWAGI, Satomi
Minamiashigara (JP)**
• **IWANAGA, Takeshi
Minamiashigara (JP)**
• **IWADATE, Yuko
Minamiashigara (JP)**
• **TAKEUCHI, Sakae
Minamiashigara (JP)**

(74) Representative: **Meissner Bolte Partnerschaft
mbB
Patentanwälte Rechtsanwälte
Postfach 86 06 24
81633 München (DE)**

(54) **CELLULOSE PARTICLE AND COSMETIC**

(57)   Cellulose particles include mother particles, containing cellulose as a principal component; and a coating layer that coats the mother particles, in which the average circularity of the cellulose particles is 0.97 or more, and the relation between carbon atom amount Cs and oxygen atom amount Os measured by X-ray photoelectron spectroscopy satisfies the formula A1: $Cs/Os \geq 2.0$.

EP 4 548 904 A1

(52) Cooperative Patent Classification (CPC): (Cont.)
C08K 2003/265

C-Sets
**C08K 3/26, C08L 1/10;**
**C08K 3/26, C08L 1/12;**
**C08L 1/10, C08L 1/286, C08K 3/26;**
**C08L 1/10, C08L 1/286, C08K 5/098;**
**C08L 1/10, C08L 1/286, C08K 5/175;**
**C08L 1/10, C08L 1/286, C08L 5/08, C08K 5/098;**
**C08L 1/10, C08L 1/286, C08L 79/02, C08K 5/098;**
**C08L 1/12, C08L 1/286, C08K 3/26**

**Description**

Background

(i) Technical Field

The present disclosure relates to cellulose particles and a cosmetic.

(ii) Related Art

Japanese Patent No. 6921293 proposes resin beads which are obtained by surface-treating core beads made of a resin, containing cellulose as a principal component, with a solid surface treatment agent and which have a volume-based cumulative 50% particle diameter of 50 $\mu$m or less, a sphericity of 0.7 to 1.0, a surface smoothness of 70% to 100%, and a crystallinity of 60% or less.

Japanese Patent No. 6694559 proposes particles containing cellulose acetate and having an average particle diameter of 80 nm or more and 100 nm or less, a sphericity of 0.7 or more and 1.0 or less, a surface smoothness of 80% or more and 100% or less, and a surface contact angle with water of 100° or more, the cellulose acetate having a total acetyl substitution degree of 0.7 or more and 2.9 or less.

Japanese Unexamined Patent Application Publication No. 2022-22947 proposes biodegradable resin particles including mother particles containing a biodegradable resin, a first layer formed on the surfaces of the mother particles and containing at least one cationic resin of polyalkyleneimine, polyallylamine, and polyvinylamine, and a second layer formed on the first layer and containing an anionic or nonionic hydrophobic compound.

Summary

Accordingly, it is an object of the present disclosure to provide cellulose particles including mother particles, containing cellulose as a principal component, and a coating layer which coats the mother particles, the cellulose particles having excellent hydrophobicity and dispersibility as compared with when the circularity is less than 0.97 and the relation between carbon atom amount Cs and oxygen atom amount Os measured by X-ray photoelectron spectrometry does not satisfy formula A1: $Cs/Os \geq 2.0.$.

According to a first aspect of the present disclosure, there are provided cellulose particles including mother particles, containing cellulose as a principal component, and a coating layer which coats the mother particles, wherein the average circularity of the cellulose particles is 0.97 or more, and the relation between carbon atom amount Cs and oxygen atom amount Os in the cellulose particles measured by X-ray photoelectron spectrometry satisfying formula A1: $Cs/Os \geq 2.0$.

According to a second aspect of the present disclosure, in the cellulose particles according to the first aspect of the present disclosure, the relation between carbon atom amount Cs and oxygen atom amount Os satisfies the formula A2: $Cs/Os \geq 4.0$.

According to a third aspect of the present disclosure, in the cellulose particles according to the first or second aspect of the present disclosure, the coating layer contains at least one coating material selected from the group consisting of a fatty acid, a fatty acid metal salt, an amino acid, and an amino acid salt.

According to a fourth aspect of the present disclosure, in the cellulose particles according to any one of the first to third aspects of the present disclosure, the coating amount of the coating layer relative to the mother particles is 2% by mass or more and 30% by mass or less.

According to a fifth aspect of the present disclosure, in the cellulose particles according to the fourth aspect of the present disclosure, the coating amount of the coating layer relative to the mother particles is 4% by mass or more and 15% by mass or less.

According to a sixth aspect of the present disclosure, the cellulose particles according to any one of the first to fifth aspects of the present disclosure further include an intermediate layer between the mother particles and the coating layer.

According to a seventh aspect of the present disclosure, in the cellulose particles according to the sixth aspect of the present disclosure, the intermediate layer contains at least one intermediate material selected from the group consisting of a polyamine compound, polyquaternium, a polysaccharide compound, and polyacrylic acid.

According to an eighth aspect of the present disclosure, there is provided a cosmetic including the cellulose particles according to any one of the first to seventh aspects of the present disclosure.

According to the first aspect of the present disclosure, there are provided cellulose particles including mother particles, containing cellulose as a principal component, and a coating layer which coats the mother particles, the cellulose particles having excellent hydrophobicity and dispersibility as compared with when the average circularity is less than 0.97 and the relation between carbon atom amount Cs and oxygen atom amount Os measured by X-ray photoelectron spectroscopy does not satisfy the formula A1: Cs/Os ≥ 2.0.

According to the second aspect of the present disclosure, there are provided cellulose particles having excellent hydrophobicity and dispersibility as compared with when the relation between carbon atom amount Cs and oxygen atom amount Os does not satisfy the formula A2: Cs/Os ≥ 4.0.

According to the third aspect of the present disclosure, there are provided cellulose particles having excellent hydrophobicity and dispersibility as compared with when the coating layer contains stearyl stearate.

According to the fourth aspect of the present disclosure, there are provided cellulose particles having excellent hydrophobicity and dispersibility as compared with when the coating amount of the coating layer relative to the mother particles is less than 2% by mass or exceeds 30% by mass.

According to the fifth aspect of the present disclosure, there are provided cellulose particles having excellent hydrophobicity and dispersibility as compared with when the coating amount of the coating layer relative to the mother particles is less than 4% by mass or exceeds 15% by mass.

According to the sixth aspect of the present disclosure, there are provided cellulose particles having excellent hydrophobicity and dispersibility as compared with when the intermediate layer is not provided between the mother particles and the coating layer.

According to the seventh aspect of the present disclosure, there are provided cellulose particles having excellent hydrophobicity and dispersibility as compared with when the intermediate layer contains carbomer.

According to the eighth aspect of the present disclosure, there is provided a cosmetic having excellent anti-sweat property and makeup anti-smudge property as compared with when cellulose particles including mother particles, containing cellulose as a principal component, and a coating layer which coats the mother particles are applied, and when the average circularity of the cellulose particles is less than 0.97 and the relation between carbon atom amount Cs and oxygen atom amount Os measured by X-ray photoelectron spectroscopy does not satisfy the formula A1: Cs/Os ≥ 2.0.

Detailed Description

An exemplary embodiment of the present disclosure is described below. The description and examples exemplify the embodiment and do not limit the scope of the embodiment.

In the numerical ranges stepwisely described in the present specification, the upper liquid value or lower limit value described in one of the numerical ranges may be replaced by the upper limit value or the lower limit value of another numerical range stepwisely described. Also, in a numerical range described in the present specification, the upper limit value or lower limit value of the numerical range may be replaced by the value described in an example.

In the exemplary embodiment, each of the components may contain plural materials corresponding to the component. In the description of the amount of each of the components in a composition, when plural materials corresponding each of the components are present in the composition, the amount represents the total amount of the plural materials present in the composition unless otherwise specified.

<Cellulose particles>

Cellulose particles according to an exemplary embodiment of the present disclosure include mother particles, containing cellulose as a principal component, and a coating layer which coats the mother particles.

The circularity of the cellulose particles according to the exemplary embodiment is 0.97 or more in terms of average circularity, and the relation between carbon atom amount Cs and oxygen atom amount Os measured by X-ray photoelectron spectrometry satisfies formula A1: Cs/Os ≥ 2.0.

The cellulose particles according to the exemplary embodiment have the configuration described above and thus become cellulose particles having excellent hydrophobicity and dispersibility. The reason for this is supposed as follows.

When the ratio (Cs/Os) between the carbon atom amount Cs and the oxygen atom amount Os of the cellulose particles is 2.0, the amount of carbon atoms present on the surfaces of particles is increased, and thus hydrophobicity is enhanced.

On the other hand, when the average circularity of the cellulose particles with the coating layer formed on the mother particles containing cellulose as a principal component is 0.97 or more, the particles come close to a spherical shape and reliability is improved. Thus, dispersibility is also enhanced.

Thus, the cellulose particles according to the exemplary embodiment are supposed to be excellent in hydrophobicity and dispersibility.

In particular, the cellulose particles according to the exemplary embodiment have the characteristics of excellent hydrophobicity and dispersibility, and thus when applied to a cosmetic, a cosmetic having excellent an anti-sweat property and makeup anti-smudge property may be obtained.

In addition, the expression "makeup smudge" represents the phenomenon that the cosmetic applied to the skin is partially gathered with time and, for example, looks like a line or the like.

(Relation between carbon atom amount Cs and oxygen atom amount Os)

In the cellulose particles according to the exemplary embodiment, the relation between carbon atom amount Cs and oxygen atom amount Os measured by X-ray photoelectron spectrometry satisfies formula A1 below. The relation preferably satisfies formula A2 below and more preferably satisfies formula A3 below.

$$\text{Formula A1: } Cs/Os \geq 2.0$$

$$\text{Formula A2: } Cs/Os \geq 4.0$$

$$\text{Formula A3: } Cs/Os \geq 5.0$$

However, the upper limit of the ratio Cs/Os is, for example, 20 or less.

When the relation between carbon atom amount Cs and oxygen atom amount Os satisfies the formula A1, that is, the ratio (Cs/Os) is 2.0 or more, the amount of carbon atoms exposed in the surfaces of the cellulose particles is increased, and thus hydrophobicity is enhanced.

A method for adjusting the ratio Cs/Os within the range described above is, for example, a method of dividing and performing plural times of surface treatments with a coating material containing carbon to form a coating layer or the like.

In the cellulose particles according to the exemplary embodiment, the relation between carbon atom amount Cs and silicon atom amount Sis measured by X-ray photoelectron spectrometry desirably satisfies formula B below. That is, the silicon atom amount Sis is desirably 0 atom% or a small amount.

$$\text{Formula B: } 0 \leq Sis/Cs \leq 0.01$$

Hydrophobicity is enhanced by carbon atoms, not silicon atoms, on the surfaces of the cellulose particles, and thus there is the advantage that surface free energy is more decreased, and the makeup anti- smudge property for sweat, sebum, and the like may be further enhanced.

- Measurement of each atom amount by X-ray photoelectron spectroscopy -

A method for measuring each atom amount (atom%) by X-ray photoelectron spectroscopy (XPS) is as follows. The measurement is performed for the cellulose particles.

The measurement is performed by using "PHI5000 Versa Probe II" manufactured by ULVAC-PHI, Inc. as a XPS measurement apparatus and monochromatized AlK$\alpha$ rays as an X-ray source with an acceleration voltage set to 15 kV. Specifically, the number of atoms (carbon atoms, silicon atoms, and oxygen atoms) is determined based on a spectrum of each of the toms (carbon atom, silicon atom, and oxygen atom) measured in an analysis region of 100 $\mu$m$\phi$ and the each of the atom amounts (carbon atom amount, silicon atom amount, and oxygen atom amount) relative to the total atom amount in the measurement region is calculated.

In measuring the surfaces of the cellulose particles, the carbon atom amount, the silicon atom amount, and the oxygen atom amount are determined as the carbon atom amount Cs, the silicon atom amount Sis, and the oxygen atom amount Os.

However, when the compound contained in the coating layer does not contain silicon atom, the silicon atom amount is 0 atom%.

(Average circularity)

The average circularity of the cellulose particles according to the exemplary embodiment is 0.97 or more, but from the viewpoint of improving hydrophobicity and dispersibility, the average circularity is preferably 0.98 or more and more preferably 0.99 or more.

In addition, the average circularity of the cellulose particles is ideally 1.

A method for adjusting the average circularity within the range described above is, for example, a method of separately performing plural surface treatments with the coating material to form the coating layer.

The circularity of cellulose particles is determined by (equivalent circle circumference)/(circumference) [(circumference of circle having the same area of a particle image)/(circumference of particle projection image)]. Specifically, the circularity is a value measured by the following method.

First, cellulose particles to be measured are collected by suction to form a flat flow, and a particle image is taken in as a static image by instantly emitting strobe light. The circularity is determined by image analysis of the particle image using a flow-type particle image analyzer (FPIA-3000 manufactured by Sysmex Corporation). The number of particles sampled for determining circularity is 3500, and an arithmetic average is calculated as the average circularity.

(Mother particle)

The mother particles are particles as an object to which the coating layer is formed and which contain cellulose as a principal component.

In this case, the expression "containing cellulose as a principal component" represents that the content of cellulose relative to the mother particles is 90% by mass or more (preferably 95% by mass or more, 98% by mass or more, or 100% by mass).

The number-average molecular weight of cellulose is preferably 37000 or more and more preferably 45000 or more.

The upper limit value of the number-average molecular weight of cellulose is not particularly limited, but may be, for example, 100000 or less.

The number-average molecular weight of cellulose is measured by a gel permeation chromatography method (differential refractometer Optilab T-rEX/manufactured by Wyatt Technology Corporation, multi-angle light scattering detector DAWN HELEOS II/manufactured by Wyatt Technology Corporation, one column each of TSKgel $\alpha$-M and $\alpha$-3000) using dimethylacetamide (0.1 M, containing lithium chloride added) as a solvent.

- Other components -

The mother particles may contain other components.

Examples of the other components include a plasticizer, a flame retardant, a compatibilizer, a release agent, a light-resistant agent, a weather-resistant agent, a coloring agent, a pigment, a modifier, a dripping inhibitor, an antistatic agent, a hydrolysis inhibitor, a filler, a reinforcing agent (glass fibers, carbon fibers, talc, clay, mica, glass flakes, milled glass, glass beads, crystalline silica, alumina, silicon nitride, aluminum nitride, boron nitride, or the like), an acid acceptor for preventing release of acetic acid (an oxide such as magnesium oxide, aluminum oxide, or the like; a metal hydroxide such as magnesium hydroxide, calcium hydroxide, aluminum hydroxide, hydrotalcite, or the like; calcium carbonate; talc; or the like), a reactive trapping agent (for example, an epoxy compound, an acid anhydride compound, carbodiimide, or the like), and the like.

The content of each of the other components relative to the total amount of the mother particles is preferably 0% by mass or more and 5% by mass or less. Herein, the expression "0% by mass" represents that the other component is not contained.

(Coating layer)

The coating layer preferably contains a coating material selected from hydrophobic compounds. Specifically, the coating layer preferably contains a coating material selected from compounds having a carboxyl group, particularly preferably contains at least one coating material selected from the group consisting of a fatty acid, a fatty acid metal salt, an amino acid, and an amino acid metal salt, and more preferably contains at least one coating material selected from the group consisting of a fatty acid metal and an amino acid metal salt. The coating material (particularly, a metal salt) causes the ratio (Cs/Os) to fall into the range described above, and thus enhances hydrophobicity of the coating layer, easily enhances circularity, and improves dispersibility.

In addition, the coating layer desirably does not contain a Si-containing compound as the coating material or even when containing a Si-containing compound, the content thereof is desirably 1% by mass or less.

- Fatty acid -

The fatty acid is a linear or branched saturated or unsaturated fatty acid. The fatty acid may be a mixture of a saturated fatty acid and an unsaturated fatty acid.

The fatty acid is preferably a fatty acid having 14 or more and 22 or less carbon atoms (preferably 14 or more and 20 or less carbon atoms). Examples of a linear fatty acid having 14 or more and 22 or less carbon atoms include behenic acid, arachidic acid, palmitic acid, stearic acid, isostearic acid, myristic acid, and the like.

- Fatty acid metal salt -

The fatty acid metal salt is a linear or branched saturated or unsaturated fatty acid metal salt. The fatty acid metal salt may be a mixture of a saturated fatty acid metal salt and an unsaturated fatty acid metal salt.

The fatty acid metal salt is, for example, a metal salt of a fatty acid having 14 or more and 22 or less carbon atoms (preferably 14 or more and 20 or less carbon atoms). Examples of a metal salt of a fatty acid having 14 or more and 22 or less carbon atoms include metal salts of stearic acid, behenic acid, palmitic acid, myristic acid, and the like.

The metal of the fatty acid metal salt is, for example, a di- or higher-valent metal.

Examples of the metal of the fatty acid metal salt include magnesium, calcium, aluminum, barium, and zinc.

- Amino acid -

The amino acid is, for example, an amino acid having 12 or more and 30 or less carbon atoms (preferably 18 or more and 24 or less carbon atoms).

Examples of the amino acid include lauroyl lysine, lauryl arginine, myristyl leucine, stearoyl glutamic acid, and the like.

- Amino acid metal salt -

The amino acid metal salt is, for example, a metal salt of an amino acid having 12 or more and 30 or less carbon atoms (preferably 18 or more and 24 or less carbon atoms).

Examples of the amino acid metal salt include metal salts of lauroyl lysine, lauryl arginine, myristyl leucine, stearoyl glutamic acid, and the like.

The metal of the amino acid metal salt is, for example, a di- or higher-valent metal.

Examples of the metal of the amino acid metal salt include magnesium, calcium, aluminum, barium, and zinc.

From the viewpoint of improving hydrophobicity and dispersibility, the coating amount of the coating layer relative to the mother particles is preferably 2% by mass or more and 30% by mass or less, more preferably 4% by mass or more and 15% by mass or less, and still more preferably 10% by mass or more and 30% by mass or less.

In this case, the content of the coating material relative to the whole of the coating layer is preferably 90% by mass or more and 100% by mass or less and more preferably 95% by mass or more and 100% by mass or less.

- Intermediate layer -

The cellulose particles according to the exemplary embodiment preferably include an intermediate layer between each of the mother particle and the coating layer. When the intermediate layer is provided, carbon atoms are aligned on the surface of the coating layer, and the ratio Cs/Os is easily brought into the range described above, thereby enhancing hydrophobicity.

The intermediate layer preferably contains at least one intermediate material selected from the group consisting of a polyamine compound, polyquaternium, a polysaccharide compound, and polyacrylic acid.

The polyamine compound is a general term of aliphatic hydrocarbons having two or more primary amino groups.

Examples of the polyamine compound include polyalkyleneimine, polyallylamine, polyvinylamine, polylysine, and the like.

From the viewpoint of improving biodegradability, the polyalkyleneimine is preferably polyalkyleneimine having a structural unit having an alkylene group having 1 or more and 6 or less carbon atoms, preferably 1 or more and 4 or less carbon atoms, and still more preferably 1 or more and 2 or less carbon atoms, and is more preferably polyethyleneimine.

Examples of the polyallylamine include homopolymers or copolymers of allylamine, an allylamine-amide sulfate salt, diallylamine, dimethylallylamine, and the like.

The polyvinylamine is, for example, polyvinylamine produced by alkali-hydrolyzing poly(N-vinylformaldehyde), and examples thereof include "PVAM-0595B" manufactured by Mitsubishi Chemical Corporation, and the like.

The polylysine may be one extracted from a natural substance, one produced using transformed microorganism, or one chemically synthesized.

Examples of the polyquaternium include polyquaternium-6, polyquaternium-7, polyquaternium-10, polyquaternium-11, polyquaternium-51, polyquaternium-61, polyquaternium-64, and the like.

The content of the polyquaternium relative to the whole of the cellulose particles is preferably 0.2% by mass or more and 2% by mass or less.

Examples of the polysaccharide compound include chitin, chitosan, carboxymethyl cellulose, and the like. Also, examples of the polysaccharide compound include polysaccharides having sulfuric acid or phosphoric acid, polysaccharides having uronic acid (for example, glucuronic acid, iduronic acid, galacturonic acid, or mannuronic acid), polysaccharides having both structures of these acids, and the like. Specific examples of polysaccharides include hyaluronic acid, gellan gum, deacylated gellan gum (DAG), rhamsan gum, diutan gum, xanthan gum, carrageenan, xanthan gum, hexuronic acid, fucoidan, pectin, pectic acid, pectinic acid, heparan sulfate, heparin, heparitin sulfate, keratosulfate, chondroitin sulfate, dermatan sulfate, rhamnan sulfate, and alginic acid.

From the viewpoint of improving hydrophobicity and dispersibility, the coating amount of the intermediate layer relative to the mother particles is preferably 0.1% by mass or more and 20% by mass or less, and more preferably 0.2% by mass or more and 2% by mass or less.

Herein, the content of the intermediate material relative to the whole of the intermediate layer is preferably 90% by mass or more and 100% by mass or less and more preferably 95% by mass or more and 100% by mass or less.

From the viewpoint of improving hydrophobicity and dispersibility, the ratio (that is, ratio = coating amount of coating layer/coating amount of intermediate layer) of the coating amount of the coating layer to the coating amount of the intermediate layer is preferably 0.05 or more and 400 or less, more preferably 1.5 or more and 150 or less, and still more preferably 5 or more and 150 or less.

- External additive -

Inorganic particles may be added to the cellulose particles according to the exemplary embodiment. When the inorganic particles are added, secondary aggregation of particles is suppressed, and thus the original characteristics of the particles are easily exhibited. Therefore, hydrophobicity and dispersibility are easily improved.

The external additive is, for example, at least one type selected from the group consisting of silicon-containing compound particles and metal oxide particles.

The silicon-containing compound particles represent particles containing silicon.

The silicon-containing compound particles may be particles containing only silicon or may be particles containing silicon and another element.

The silicon-containing compound particles are preferably silica particles. The silica particles may be particles containing silica, that is, $SiO_2$, as a principal component and may be crystalline or amorphous. In addition, the silica particles may be particles produced using a silicon compound, such as water glass, alkoxysilane, or the like, as a raw material or may be particles produced by grinding quartz.

An oxide of a metal other than silicon may be applied as a metal oxide
Examples of the metal oxide include zinc oxide, magnesium oxide, iron oxide, aluminum oxide, and the like.

From the viewpoint of texture (specifically, touch), the volume-average particle diameter of the external additive is preferably 1 nm or more and 100 nm or less and more preferably 5 nm or more and 30 nm or less.

The volume-average particle diameter of the external additive is measured by the same method as for the volume-average particle diameter of cellulose.

The external addition amount of the external additive relative to the cellulose particles (cellulose particles without containing the external additive externally added) is preferably 0.1% by mass or more and 2% by mass or less.

(Volume-average particle diameter)

The volume-average particle diameter of the cellulose particles according to the exemplary embodiment is preferably 1 μm or more and 100 μm or less, more preferably 2 μm or more and 20 μm or less, and still more preferably 4 μm or more and 10 μm or less.

When the volume-average particle diameter of the cellulose particles according to the exemplary embodiment is 1 μm or more and 100 μm or less, the particles are of appropriate size, and hydrophobicity and dispersibility are improved.

The volume-average particle diameter of the cellulose particles is measured as follows.

Particle diameters are measured by using LS particle size distribution analyzer "Beckman Coulter LS13 320 (manufactured by Beckman Coulter Inc.)", a volume-based cumulative distribution of particle diameters is drawn from the small-diameter side, and the particle diameter at 50% cumulation is determined as the volume-average particle diameter.

<Method for producing cellulose particles>

A method for producing the cellulose particles according to the exemplary embodiment include, for example, producing mother particles containing cellulose as a principal component (referred to as "production of cellulose particles (mother particles)" hereinafter) and coating the mother particles with a coating material, and if required, an intermediate material (referred to as "Formation of intermediate layer and coating layer" hereinafter)

Specifically, the method for producing the cellulose particles according to the exemplary embodiment is, for example, as follows.

- Production of mother particles -

(1) First, cellulose acylate is dissolved in a water-soluble organic solvent A to prepare a cellulose acylate solution A.

(2) Next, the cellulose acylate solution A is added to a calcium carbonate dispersed liquid containing calcium carbonate dispersed in water and stirred to prepare a cellulose acylate solution B.

(3) Next, the cellulose acylate solution B is added to a mixed solution of carboxymethyl cellulose, a water-soluble organic solvent B, and water and stirred at a high speed to prepare a cellulose acylate solution C.

(4) Next, sodium hydroxide is added to the cellulose acylate solution C, then the water-soluble organic solvents A and B are removed by heating the cellulose acylate solution C, and then hydrochloric acid is added the residue to form cellulose acylate particles. The cellulose acylate particles are filtered off, and the filtered-off cellulose acylate particles are dispersed in water to prepare a cellulose acylate particle dispersed liquid.

(5) Next, sodium hydroxide is added to the cellulose acylate particle dispersed liquid, and then the cellulose acylate particle dispersed liquid is heated in a weak alkaline environment and stirred, and the cellulose acylate particles are saponified to prepare a mother particle suspension.

(6) Next, the pH of the suspension is adjusted to near neutral (for example, within a range of 6.5 or more and 7 or less) by adding hydrochloric acid to the mother particle suspension, and then the mother particles are repeatedly filtered off and washed with pure water. Then, after the electric conductivity of the filtrate reaches 10 μs/cm or less, the filtered-off mother particles are dried.

The cellulose acylate is a cellulose derivative produced by substituting (acylating) at least one hydroxyl group of cellulose with an aliphatic acyl group. Specifically, the cellulose acylate is a cellulose derivative produced by substituting at least one hydroxyl group of cellulose with $-CO-R^{AC}$ ($R^{AC}$ represents an aliphatic hydrocarbon group).

The water-soluble organic solvent A is a solvent in which 0.1% by mass or more and 10% by mass or less of water is dissolved in the solvent at 25°C, and examples thereof ethyl acetate, butyl acetate, and the like.

The water-soluble organic solvent B is a solvent in which 0.1% by mass or more and 10% by mass or less of water is dissolved in the solvent at 25°C, and examples thereof methyl ethyl ketone, acetone, and the like.

- Formation of intermediate layer and coating layer -

First, a water dispersion liquid containing the mother particles dispersed therein is prepared. Before the water dispersion liquid is prepared, the mother particles are desirably washed with an acid.

Next the water dispersion liquid containing the mother particles dispersed therein is mixed with an aqueous solution containing the intermediate material which constitutes the intermediate layer. Thus, for example, hydroxyl groups of cellulose contained in the mother particles react with an amine site, a carboxyl group, an amino group, etc. of a compound constituting the intermediate layer, and hydroxyl groups form hydrogen bonds, forming the intermediate layer. In addition, the operation of forming the intermediate layer is performed plural times.

However, when the intermediate layer is not formed, this operation is not performed.

Then, the water dispersion liquid containing the mother particles dispersed therein, on which the intermediate layer is formed, is heated, and then the coating material which constitutes the coating layer is added and stirred. Thus, the coating layer is formed. The operation of forming the coating layer is desirably performed plural times.

In this case, when the coating layer is formed, the coating amount of the coating layer is controlled by the heating time, the amount of the coating material added, the addition time of the coating material, and the stirring time after the addition of the coating material.

Then, the mother particles having the coating layer formed thereon are removed from the mixed solution. The mother particles having the coating layer formed thereon are removed by, for example, filtering the mixed solution. The removed mother particles having the coating layer formed thereon are desirably washed with water. Therefore, the unreacted intermediate material and coating material may be removed. Then, the mother particles having the coating layer formed thereon are dried to obtain the cellulose particles according to the exemplary embodiment.

- External addition -

The external additive may be added to the resultant cellulose particles.

The external addition includes, for example, treatment of adding the external additive to the cellulose particles by using a mixing mill, a V-type blender, a Henschel mixer, Loedige mixer, or the like.

<Application>

Examples of application of the cellulose particles according to the exemplary embodiment include particles of a cosmetic, a rolling agent, an abrasive, a scrub agent, a display spacer, a bead forming material, light-diffusing particles, a resin reinforcing agent, a refractive index controlling agent, a biodegradation accelerator, a fertilizer, water-absorbing particles, toner particles, and anti-blocking particles.

<Cosmetic>

A cosmetic according to an exemplary embodiment of the present disclosure is a cosmetic containing the cellulose particles according to the exemplary embodiment.

Examples of the cosmetic according to the exemplary embodiment include cosmetics for base make (for example, a makeup base, concealer, foundation, face powder, and the like), cosmetics for makeup (for example, lipstick, gloss, lipliner, cheek, eyeshadow, eyeliner, mascara, eyebrow, nail, a cosmetic for nail care, and the like), cosmetics for skincare (for example, a facial cleanser, a cleanser, a lotion, a milky lotion, a beauty serum, a pack, a facemask, a cosmetic for eyes and mouth, and the like), and the like.

In particular, the cosmetic according to the exemplary embodiment has a high anti-sweat property and makeup anti-smudge property and is thus preferably a cosmetic for makeup.

[EXAMPLES]

Examples are described below, but the present disclosure is not limited to these examples. In description below, all "parts" and "%" are on mass basis unless otherwise specified.

<Preparation of each material>

Each of the materials described below is prepared.

<EXAMPLES 1 to 17 and COMPARATIVE EXAMPLES 1 to 3>

- Formation of mother particles -

First, 130 parts of DAC ""L-50" manufactured by Daicel Corporation, cellulose diacetate, weight-average polymerization degree: 570" as cellulose acylate is completely dissolved in 870 parts of ethyl acetate. The resultant solution is added to a dispersion liquid prepared by dispersing 50 parts of calcium carbonate in 500 parts of pure water and stirred for 3 hours. The resultant mixture is added to a dispersion liquid prepared by dispersing 4 parts of carboxymethyl cellulose and 200 parts of methyl ethyl ketone in 600 parts of pure water and stirred for 5 minutes by a high-speed emulsification machine. Then, 10 parts of sodium hydroxide is added to the resultant mixture and stirred at 80°C for 3 hours, removing ethyl acetate and methyl ethyl ketone. Then, 10 parts of dilute hydrochloric acid is added to the residue, dissolving calcium carbonate. The residue is filtered and again dispersed in pure water to obtain a slurry of cellulose acylate particles.

Then, 17.5 parts of a 20% aqueous sodium hydroxide solution is added to 500 parts of the resultant slurry of cellulose acylate particles (solid content: 10%) and stirred at 30°C for 6 hours to saponify the cellulose acylate particles. Then, hydrochloric acid is dropped to the slurry until pH of the slurry is 7. Then, the slurry is filtered, and the filtered-off material is washed with an excessive amount of pure water. The filtration and washing are repeated until the electric conductivity of the filtrate becomes 10 μs/cm or less. The final resultant cake-like filtered-off material is filtered, and the filtered-off material is freeze-dried to obtain saponified cellulose mother particles.

- Surface treatment -

The cake-like cellulose mother particles obtained by repeating filtration and washing until the electric conductivity of the filtrate is 10 μs/cm or less are re-slurried with pure water, obtaining a mother particle slurry.

Then, an intermediate material, whose type and amount are shown in Table 1, is added to 500 parts of the mother particle slurry (solid content: 10%), and stirring is continued for a stirring time shown in Table 1 while 35°C is maintained. This treatment is performed the number of times (in the table, the number of treatment times) shown in Table 1. Thus, the intermediate layer with a coating amount shown in Table 1 is formed.

Next, the mother particle slurry with the intermediate layer formed thereon is heated to a temperature of 80°C, and then a coating material, whose type and amount are shown in Table 1, is added and stirred for a stirring time shown in Table 1. This treatment is performed the number of times (in the table, the number of times of division) shown in Table 1. Thus, the coating layer with a coating amount shown in Table 1 is formed on the intermediate layer on the mother particles.

Then, the mother particles having the intermediate layer and the coating layer formed thereon are repeatedly filtered off and washed and washed until the electric conductivity of the filtrate is 10 μs/cm or less. After washing, the resultant cake is dried to obtain cellulose particles having the intermediate layer and the coating layer.

Also, the resultant particles are stirred for 2 hours with a number of rotations of 4000 min$^{-1}$ by using FM mixer (FM40, manufactured by Nippon Coke & Engineering Co., Ltd.) while the temperature of the mixer is maintained at 25°C, tanning the surface of the coating layer.

As shown by "-" in Table 1, the cellulose particles are formed without forming the intermediate layer or forming the intermediate layer and the coating layer in some of the examples.

<COMPARATIVE EXAMPLES 4 and 5>

Commercial cellulose particles described below are used as cellulose particles in each of Comparative Examples 4 and 5.

Comparative Example 4: "S-STM CELLULOBEADS D-5" manufactured by Daito Kasei Kogyo Co., Ltd.

Comparative Example 5: "OTS-0.5A CELLULOBEADS D-10" manufactured by Daito Kasei Kogyo Co., Ltd.

<Evaluation>

(Particle properties)

The particle properties of the cellulose particles obtained in each of the examples are measured according to the methods described above.

· Ratio (Cs/Os) between carbon atom amount Cs and oxygen atom amount Os measured by X-ray photoelectron spectroscopy
· Average circularity
· Volume-average particle diameter of cellulose particles

(Artificial sweat contact angle)

A liquid foundation is obtained by a known method using the cellulose particles of each of the examples according to the prescription shown in Table 2.

The liquid foundation is coated on an artificial skin and allowed to stand for 1 hour at room temperature. The artificial sweat is dropped using a contact angle measuring device, and the artificial sweat contact angle is measured. The evaluation criteria are as follows.

A: $100° \leq$ contact angle
B: $90° \leq$ contact angle $\leq 100°$
C: Contact angle $< 90°$

(Makeup smudge)

A liquid foundation is obtained by a known method using the cellulose particles of each of the examples according to the prescription shown in Table 2.

Five g of the liquid foundation is placed on the back of the hand of each of 10 monitors and spread thereon. After the passage of 8 hours, points are given to makeup smudge on a scale of 0 to 10, 10 being the best and 0 being the worst. The arithmetic average of the points of ten monitors is evaluated.

[Table 1-1]

| | Resin type before saponification | Resin type after saponification | Intermediate layer | | | | | Coating layer | | | | |
|---|---|---|---|---|---|---|---|---|---|---|---|---|
| | | | Intermediate material | Amount of intermediate material added / % (relative to mother particle) | Number of times of division of intermediate material / times | Stirring time of intermediate material / h | Coating amount / % (relative to mother particle) | Coating material | Amount of coating material added / % (relative to mother particle) | Number of times of division of coating material / times | Stirring time of coating material / h | Coating amount / % (relative to mother particle) |
| Example 1 | DAC | Cellulose | Chitosan | 0.34 | 3 | 2 | 1 | Aluminum stearoylglutamate | 2.67 | 3 | 5 | 8 |
| Example 2 | DAC | Cellulose | Chitosan | 0.34 | 3 | 2 | 1 | Aluminum stearate | 2.67 | 3 | 5 | 8 |
| Example 3 | DAC | Cellulose | Chitosan | 0.34 | 3 | 2 | 1 | Calcium stearate | 2.67 | 3 | 5 | 8 |
| Example 4 | DAC | Cellulose | Polyethyleneimine | 0.34 | 3 | 2 | 1 | Behenic acid | 2.67 | 3 | 5 | 8 |
| Example 5 | DAC | Cellulose | Polyethyleneimine | 0.34 | 3 | 2 | 1 | Stearoylglutamic acid | 2.67 | 3 | 5 | 8 |
| Example 6 | DAC | Cellulose | Polyethyleneimine | 0.34 | 3 | 2 | 1 | Lauroyl lysine | 2.67 | 3 | 5 | 8 |
| Example 7 | DAC | Cellulose | Chitosan | 0.34 | 3 | 2 | 1 | Aluminum stearoylglutamate | 12 | 3 | 5 | 36 |
| Example 8 | DAC | Cellulose | Chitosan | 0.34 | 3 | 2 | 1 | Aluminum stearoylglutamate | 10 | 3 | 5 | 30 |
| Example 9 | DAC | Cellulose | Chitosan | 0.34 | 3 | 2 | 1 | Aluminum stearoylglutamate | 5 | 3 | 5 | 15 |
| Example 10 | DAC | Cellulose | - | - | - | - | - | Aluminum stearoylglutamate | 2.67 | 3 | 5 | 8 |
| Example 11 | DAC | Cellulose | Chitosan | 0.5 | 2 | 3 | 1 | Aluminum stearoylglutamate | 4 | 2 | 7.5 | 8 |
| Example 12 | DAC | Cellulose | Chitosan | 0.34 | 3 | 0.5 | 1 | Aluminum stearoylglutamate | 2.67 | 3 | 1 | 8 |
| Example 13 | DAC | Cellulose | Chitosan | 0.5 | 2 | 3 | 1 | Aluminum stearoylglutamate | 2 | 2 | 7.5 | 4 |

(continued)

| | Resin type before saponification | Resin type after saponification | Intermediate layer | | | | | Coating layer | | | | |
|---|---|---|---|---|---|---|---|---|---|---|---|---|
| | | | Intermediate material | Amount of intermediate material added / % (relative to mother particle) | Number of times of division of intermediate material / times | Stirring time of intermediate material / h | Coating amount / % (relative to mother particle) | Coating material | Amount of coating material added / % (relative to mother particle) | Number of times of division of coating material / times | Stirring time of coating material / h | Coating amount / % (relative to mother particle) |
| Example 14 | DAC | Cellulose | Chitosan | 0.34 | 3 | 0.5 | 1 | Aluminum stearoylglutamate | 0.67 | 3 | 1 | 2 |
| Example 15 | DAC | Cellulose | Chitosan | 0.34 | 3 | 0.5 | 1 | Aluminum stearoylglutamate | 0.34 | 3 | 1 | 1 |
| Example 16 | DAC | Cellulose | Chitosan | 0.34 | 3 | 2 | 1 | Stearyl stearate | 2.67 | 3 | 5 | 8 |
| Example 17 | DAC | Cellulose | Carbomer | 0.34 | 3 | 2 | 1 | Aluminum stearoylglutamate | 2.67 | 3 | 5 | 8 |
| | | | | | | | | | | | | |
| Comparative Example 1 | DAC | Cellulose | - | - | - | - | - | - | - | - | - | - |
| Comparative Example 2 | DAC | Cellulose | - | - | - | - | - | Aluminum stearoylglutamate | 0.5 | 1 | 0.1 | 0.5 |
| Comparative Example 3 | DAC | Cellulose | Chitosan | 0.5 | 1 | 0.1 | 0.5 | Aluminum stearoylglutamate | 0.5 | 1 | 0.1 | 0.5 |
| Comparative Example 4 | Daito Kasei Kogyo Co., Ltd [S-STM CELLULOBEADS D-5] | | | | | | | | | | | |
| Comparative Example 5 | Daito Kasei Kogyo Co., Ltd [OTS-0.5A CELLULOBEADS D-10] | | | | | | | | | | | |

[Table 1-2]

| | Particle properties | | Evaluation | |
|---|---|---|---|---|
| | XPS Cs/Os ratio | Circularity | Artificial sweat contact angle | Makeup anti-smudge property |
| Example 1 | 4.8 | 0.986 | A | 9 |
| Example 2 | 6.3 | 0.986 | A | 9 |
| Example 3 | 8.2 | 0.99 | A | 9 |
| Example 4 | 7.1 | 0.984 | A | 8 |
| Example 5 | 4.3 | 0.982 | A | 8 |
| Example 6 | 4.6 | 0.98 | A | 8 |
| Example 7 | 5.8 | 0.97 | B | 6 |
| Example 8 | 5.8 | 0.971 | B | 7 |
| Example 9 | 5.5 | 0.98 | A | 8 |
| Example 10 | 3.6 | 0.972 | B | 6 |
| Example 11 | 3.9 | 0.98 | A | 8 |
| Example 12 | 3.5 | 0.98 | A | 8 |
| Example 13 | 3.1 | 0.988 | A | 8 |
| Example 14 | 2.6 | 0.99 | B | 7 |
| Example 15 | 2.2 | 0.989 | B | 6 |
| Example 16 | 2.5 | 0.98 | B | 6 |
| Example 17 | 3.8 | 0.973 | B | 6 |
| | | | | |
| Comparative Example 1 | 1.2 | 0.99 | C | 2 |
| Comparative Example 2 | 1.5 | 0.985 | C | 3 |
| Comparative Example 3 | 1.9 | 0.988 | C | 4 |
| Comparative Example 4 | 8.1 | 0.967 | C | 5 |
| Comparative Example 5 | 1.8 | 0.99 | C | 4 |

[Table 2]

| Prescription | Compound | Product name (Manufacturer) | Parts by mass |
|---|---|---|---|
| Particle | Particle | Cellulose particle of each example | 10 |
| Prescription of compounds other than particle | Propylene glycol | Propylene Glycol JSQI (Dow Toray°Co., Ltd.) | 5 |
| | Bentonite | OVWIL BR (Mizusawa Industrial Chemicals, Ltd.) | 1 |
| | Triethanolamine | Triethanolamine 99% (Dow Toray°Co., Ltd.) | 1 |
| | Stearic acid | NAA172 (NOF Corporation) | 3 |
| | Stearyl alcohol | NAA45 (NOF Corporation) | 1 |
| | Fluid paraffin | Moresco Violence (MORESCO) | 8 |
| | Isopropyl myristate | IPM-R (NOF Corporation) | 5 |

(continued)

| Prescription | Compound | Product name (Manufacturer) | Parts by mass |
|---|---|---|---|
| | Vaseline | Nomucoat W (Nisshin OilliO Ltd.) | 2 |
| | Monoglyceride stearate | Excel 84 (Kao Chemical Co., Ltd.) | 2 |
| | POE(20) Stearyl ether | EMALEX602 (Nihon Emulsion Co., Ltd.) | 1 |
| | Titanium oxide | MKR-1 (Sakai Chemical Industry Co., Ltd.) | 8 |
| | Titanium oxide | BERACLAY 20061AMAZONIAN WHITE CLAY (BERECA) | 5 |
| | Iron oxide | C33-128 SunCROMA RED Iron Oxide (Sun Chemical) | 0.5 |
| | Preservative | POTIPHEN HD (Ashland°Japan Inc.) | 0.5 |
| | Fragrance | Bisabolol rac. (BASF Japan Ltd.) | 0.3 |
| | Purified water | | 46.5 |
| Total | | | 100 |

The results described above indicate that the cellulose particles of the examples are excellent in terms of anti-sweat resistance and makeup anti-smudging resistance as compared with the cellulose particles of the comparative examples.

Therefore, it is also found that the cellulose particles of the examples are excellent in terms of hydrophobicity and dispersibility as compared with the cellulose particles of the comparative examples.

The foregoing description of the exemplary embodiments of the present disclosure has been provided for the purposes of illustration and description. It is not intended to be exhaustive or to limit the disclosure to the precise forms disclosed. Obviously, many modifications and variations will be apparent to practitioners skilled in the art. The embodiments were chosen and described in order to best explain the principles of the disclosure and its practical applications, thereby enabling others skilled in the art to understand the disclosure for various embodiments and with the various modifications as are suited to the particular use contemplated. It is intended that the scope of the disclosure be defined by the following claims and their equivalents.

Appendix

(((1))) Cellulose particles including mother particles, containing cellulose as a principal component, and a coating layer which coats the mother particles, in which the average circularity of the cellulose particles is 0.97 or more and the relation between carbon atom amount Cs and oxygen atom amount Os in the cellulose particles measured by X-ray photoelectron spectroscopy satisfies the formula A1: $Cs/Os \geq 2.0$.

(((2))) The cellulose particles described in (((1))), in which the relation between carbon atom amount Cs and oxygen atom amount Os satisfies the formula A2: $Cs/Os \geq 4.0$.

(((3))) The cellulose particles described in (((1))) or (((2))), in which the coating layer contains at least one coating material selected from the group consisting of a fatty acid, a fatty acid metal salt, an amino acid, and an amino acid salt.

(((4))) The cellulose particles described in any one of (((1))) to (((3))), in which the coating amount of the coating layer relative to the mother particles is 2% by mass or more and 30% by mass or less.

(((5))) The cellulose particles described in (((4))), in which the coating amount of the coating layer relative to the mother particles is 4% by mass or more and 15% by mass or less.

(((6))) The cellulose particles described in any one of (((1))) to (((5))), further including an intermediate layer between the mother particles and the coating layer.

(((7))) The cellulose particles described in (((6))), in which the intermediate layer contains at least one intermediate material selected from the group consisting of a polyamine compound, polyquaternium, a polysaccharide compound, and polyacrylic acid.

(((8))) A cosmetic including the cellulose particles described in any one of (((1))) to (((7))).

The effects of the aspects described above are as follows.

According to the disclosure relating to (((1))), there are provided cellulose particles including mother particles, containing cellulose as a principal component, and a coating layer which coats the mother particles, the cellulose particles having excellent hydrophobicity and dispersibility as compared with when the average circularity is less than 0.97 and the relation between carbon atom amount Cs and oxygen atom amount Os measured by X-ray photoelectron spectroscopy does not satisfy the formula A1: $Cs/Os \geq 2.0$.

According to the disclosure relating to (((2))), there are provided cellulose particles having excellent hydrophobicity and dispersibility as compared with when the relation between carbon atom amount Cs and oxygen atom amount Os does not satisfy the formula A2: $Cs/Os \geq 4.0$.

According to the disclosure relating to (((3))), there are provided cellulose particles having excellent hydrophobicity and dispersibility as compared with when the coating layer contains stearyl stearate.

According to the disclosure relating to (((4))), there are provided cellulose particles having excellent hydrophobicity and dispersibility as compared with when the coating amount of the coating layer relative to the mother particles is less than 2% by mass or exceeds 30% by mass.

According to the disclosure relating to (((5))), there are provided cellulose particles having excellent hydrophobicity and dispersibility as compared with when the coating amount of the coating layer relative to the mother particles is less than 4% by mass or exceeds 15% by mass.

According to the disclosure relating to (((6))), there are provided cellulose particles having excellent hydrophobicity and dispersibility as compared with when the intermediate layer is not provided between the mother particles and the coating layer.

According to the disclosure relating to (((7))), there are provided cellulose particles having excellent hydrophobicity and dispersibility as compared with when the intermediate layer contains carbomer.

According to the disclosure relating to (((8))), there is provided a cosmetic having excellent anti-sweat property and makeup anti-smudge property as compared with when cellulose particles including mother particles, containing cellulose as a principal component, and a coating layer which coats the mother particles are applied, and when the average circularity of the cellulose particles is less than 0.97 and the relation between carbon atom amount Cs and oxygen atom amount Os measured by X-ray photoelectron spectroscopy does not satisfy the formula A1: $Cs/Os \geq 2.0$.

## Claims

1. Cellulose particles comprising:

    mother particles, containing cellulose as a principal component; and
    a coating layer that coats the mother particles,
    wherein the average circularity of the cellulose particles is 0.97 or more; and
    the relation between carbon atom amount Cs and oxygen atom amount Os in the cellulose particles measured by X-ray photoelectron spectroscopy satisfies the formula A1: $Cs/Os \geq 2.0$.

2. The cellulose particles according to claim 1, wherein the relation between carbon atom amount Cs and oxygen atom amount Os satisfies the formula A2: $Cs/Os \geq 4.0$.

3. The cellulose particles according to claim 1 or 2, wherein the coating layer contains at least one coating material selected from the group consisting of a fatty acid, a fatty acid metal salt, an amino acid, and an amido acid salt.

4. The cellulose particles according to any one of claims 1 to 3, wherein the coating amount of the coating layer relative to the mother particles is 2% by mass or more and 30% by mass or less.

5. The cellulose particles according to claim 4, wherein the coating amount of the coating layer relative to the mother particles is 4% by mass or more and 15% by mass or less.

6. The cellulose particles according to any one of claims 1 to 5, further comprising an intermediate layer between the mother particles and the coating layer.

7. The cellulose particles according to claim 6, wherein the intermediate layer contains at least one intermediate material selected from the group consisting of a polyamine compound, polyquaternium, a polysaccharide compound, and polyacrylic acid.

8.  A cosmetic comprising the cellulose particles according to any one of claims 1 to 7.

**EUROPEAN SEARCH REPORT**

Application Number

EP 24 19 5519

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/250241 A1 (YAO KENJI [JP] ET AL) 10 August 2023 (2023-08-10) <br> * paragraphs [0005], [0119], [0153] - [0158] * <br> * example 1; tables 1-1 * <br> * examples 65-76; tables 1-4 * <br> ----- | 1-8 | INV. <br> A61K8/02 <br> A61K8/73 <br> C09D105/08 <br> C09D133/08 <br> C09D179/00 <br> C09D191/00 |
| E | EP 4 438 669 A1 (FUJIFILM BUSINESS INNOVATION CORP [JP]) 2 October 2024 (2024-10-02) <br> * paragraphs [0006], [0125] - [0127] * <br> * examples 1-33, 36-41; tables 1-1 * <br> * claims 1-7, 11 * <br> ----- | 1-8 | C09D179/02 |

TECHNICAL FIELDS
SEARCHED        (IPC)

C08K
C09J
A61Q
C08F
A61K
C09D

The present search report has been drawn up for all claims

| Place of search | Date of completion of the search | Examiner |
|---|---|---|
| Munich | 6 February 2025 | Lartigue, M |

## ANNEX TO THE EUROPEAN SEARCH REPORT
## ON EUROPEAN PATENT APPLICATION NO.

EP 24 19 5519

This annex lists the patent family members relating to the patent documents cited in the above-mentioned European search report.
The members are as contained in the European Patent Office EDP file on
The European Patent Office is in no way liable for these particulars which are merely given for the purpose of information.

06-02-2025

| Patent document cited in search report | | Publication date | Patent family member(s) | | Publication date |
|---|---|---|---|---|---|
| US 2023250241 | A1 | 10-08-2023 | CN | 116574311 A | 11-08-2023 |
| | | | JP | 2023115643 A | 21-08-2023 |
| | | | US | 2023250241 A1 | 10-08-2023 |
| EP 4438669 | A1 | 02-10-2024 | EP | 4438669 A1 | 02-10-2024 |
| | | | US | 2024325258 A1 | 03-10-2024 |

EPO FORM P0459

For more details about this annex : see Official Journal of the European Patent Office, No. 12/82

**REFERENCES CITED IN THE DESCRIPTION**

**Patent documents cited in the description**

- JP 6921293 B
- JP 6694559 B

- JP 2022022947 A